# DEMANDE DE BREVET EUROPEEN

(11) **EP 1 256 800 A2**
(43) Date de publication de la demande: **13.11.2002**
(21) Numéro de dépôt: 02291173.9
(22) Date de dépôt: 10.05.2002
(51) Int. Cl.: G01N 33/18, C12Q 1/02

(54) **Test embryolarvaire de bivalves marins in situ**

(30) Priorité: 10.05.2001 FR 0106163
(71) Demandeur: INSTITUT FRANCAIS DE RECHERCHE POUR L'EXPLOITATION DE LA MER (IFREMER), 92138 Issy-les-Moulineaux Cedex (FR)
(72) Inventeur: Geffard, Olivier, Patio de Meyran, 33470 Gujan Mestras (FR); His, Edouard, 33470 Le Teich (FR)
(74) Mandataire: Vialle-Presles, Marie José

(57) **Abrégé**

L'invention est relative à un procédé de détermination de la qualité de l'eau de mer, par immersion d'un récipient contenant des oeufs de bivalves marins à l'endroit où l'on souhaite déterminer la qualité de l'eau, et détection des anomalies présentées par les larves développées à partir desdits oeufs.

## Description

L'invention est relative à des bioessais *in situ* permettant de déterminer la qualité biologique de l'eau de mer.

Les bioessais, faisant appel à des organismes vivants pour détecter la présence d'agents polluants dans le milieu naturel, sont de plus en plus utilisés, notamment pour évaluer la qualité de l'eau en zone littorale .

Pour déterminer la qualité biologique de l'eau de mer on emploie ainsi fréquemment en laboratoire des mollusques bivalves marins, en particulier des huîtres du genre *Crassostrea* et des moules du genre *Mytilus.* Des bioessais utilisant des animaux à différents stades de leur développement, de l'embryon jusqu'à l'animal adulte, ont ainsi été proposés. L'utilisation d'embryons ou de larves de mollusques bivalves, qui a été initialement suggérée par PRYTHERCH (Report of the U.S. Commissioner of Fisheries for the fiscal year 1923, appendix XI, document 961, 1924), puis développée par WOELKE (Proceedings of the Northwest Symposium on Water Pollution Research, US Dept, HEWPHS, Portland, Oregon, 113-123, 1961 ; Water Quality Criteria, ASTM STP 416, American Society for Testing and Materials, 112-120, 1967 ; Washington Department of Fisheries Technical Report, 9, 1-93, 1972) et DIMICK et BREESE (Proc. 12^{th} Pacific Northwest Industrial Waste Conf., Univ. Wash. College Engng. Dpt. Civil Engng., 165-175, 1965), fait actuellement l'objet d'un grand intérêt : les embryons et larves, qui sont beaucoup plus sensibles que les animaux adultes aux influences du milieu extérieur, ont été utilisés pour mettre au point des tests embryo-larvaires très sensibles qui ont permis d'évaluer l'effet de très nombreux micropolluants (polluants organiques ou inorganiques divers, effluents industriels, etc.).

A l'heure actuelle, les tests embryo-larvaires de bivalves marins sont conduits en laboratoire, sur des échantillons d'eau de mer préalablement prélevés sur le site que l'on souhaite étudier, et sont parfaitement standardisés (American society for testing and Materials. "Standard guide for conducting static acute toxicity tests starting with embryos of four species of saltwater bivalve molluscs". *ASTM*, E 724-89, Philadelphia, PA, 334-351, 1989; pour revue cf. également HIS *et al.* (Adv. Mar. Biol., 37, 1-178, 1999). Des oeufs fertilisés d'un mollusque bivalve, en nombre défini, sont placés dans des récipients contenant des échantillons de l'eau de mer à tester, et maintenus dans des conditions définies jusqu'à ce que les larves issues de ces oeufs aient atteint le stade véligère (larves D), soit environ 24 heures dans le cas des larves d'huître, 48 heures dans le cas des larves de moule. A l'issue de cette incubation, les larves sont récupérées et sont examinées pour détecter les anomalies induites par la présence d'agents toxiques.

Les résultats sont comparés avec ceux relevés pour les larves développées dans une eau de mer témoin, dépourvue de polluants.

Les tests effectués en laboratoire présentent toutefois l'inconvénient de ne pas toujours rendre compte des conditions environnementales naturelles au site de prélèvement (variations des conditions de milieu en fonction de la marée et conditions thermiques non constantes, par exemple). Les épisodes toxiques peuvent être sous-estimés par des prélèvements ponctuels et certains toxiques volatiles ou labiles peuvent disparaître lors de l'acheminement des échantillons au laboratoire.

Pour éliminer les artéfacts liés au prélèvement et au stockage des échantillons, et mieux simuler les conditions naturelles de milieu, il a été proposé d'utiliser des tests *in situ*.

Des bioessais *in situ* utilisant des larves d'arthropodes ont ainsi été proposés pour mesurer la qualité des eaux douces (CRANE *et al.*, Wat. Res., 29, 2441-2448, 1995 ; MEREGALLI *et al.*, Ecotoxicol. Environ. Safety., 47(3), 231-239, 2000 ; PEREIRA *et al.*, Ecotoxicol. Environ. Safety. 47(1), 27-39, 2000).

Dans le cas des mollusques bivalves, seuls des animaux adultes (SALAZAR et SALAZAR, Environmental Toxicity and Risk Assessment, vol. 3, American Society for Testing and Material, Philadelphia PA, 216-241, 1995) ou juvéniles (WARRIN *et al.*, J. North. Am. Benthol. Soc., 14, 341-346, 1995) ont été utilisés pour des bioessais *in situ*.

En effet, du fait de la grande sensibilité des embryons et des larves à l'ensemble des facteurs environnementaux, on supposait que la survie dans les conditions de test *in situ*, où interviennent de nombreux facteurs potentiels de mortalité autres que la pollution, ne serait pas suffisante pour permettre une évaluation de la qualité biologique de l'eau.

Or, les Inventeurs ont maintenant constaté que, de manière inattendue, les tests embryo-larvaires de bivalves marins pouvaient être effectués sur site, et permettaient d'évaluer de façon fiable et reproductible la qualité biologique de l'eau de mer.

La présente invention a en conséquence pour objet un procédé de détermination de la qualité biologique de l'eau de mer, caractérisé en ce qu'il comprend :
a) le transfert d'un nombre défini d'oeufs fertilisés d'un mollusque bivalve marin dans un récipient d'exposition enfermant lesdits oeufs et permettant le passage de l'eau de mer ;
b) l'immersion dudit récipient d'exposition à l'endroit où l'on souhaite déterminer la qualité de l'eau, et son maintien en immersion pendant un temps suffisant pour permettre le développement desdits oeufs jusqu'au stade véligère ;
c) la récupération des larves, et la détermination du taux de mortalité et/ou du taux d'anomalies présentes chez lesdites larves.

De préférence, le procédé conforme à l'invention comprend en outre la réalisation d'au moins un essai témoin, dans lequel on détermine le taux de mortalité et/ou le taux d'anomalies chez des larves véligères développées dans les mêmes conditions, dans une eau de mer non polluée.

Cet essai témoin peut par exemple être effectué en mettant en oeuvre les étapes a), b), et c) définies ci-dessus, l'immersion de l'étape b) étant effectuée à un endroit dépourvu de pollution.

De préférence, cet essai témoin sera effectué en mettant en oeuvre les étapes a') b') et c') suivantes :
a') le transfert d'un nombre défini d'oeufs fertilisés d'un mollusque bivalve marin dans un récipient témoin étanche, contenant une eau de mer non polluée ;
b') l'immersion dudit récipient témoin au même endroit que le récipient d'exposition, et son maintien en immersion pendant le même temps ;
c') la récupération des larves dudit récipient témoin, et la détermination du taux de mortalité et/ou du taux d'anomalies présentes chez lesdites larves.

Ce mode de mise en oeuvre permet de contrôler que le récipient témoin et le récipient d'exposition demeurent à la même température, et également que l'eau du récipient témoin demeure dépourvue de pollution pendant toute la durée de l'immersion.

Pour la mise en oeuvre du procédé conforme à l'invention, le transfert des oeufs fertilisés dans le récipient d'exposition et le récipient témoin, et l'immersion de ceux-ci s'effectuent de préférence dans les 2 heures qui suivent, et de manière tout à fait préférée dans l'heure qui suit la fertilisation.

L'immersion s'effectue à une profondeur comprise entre 20 cm et 20 mètres, de préférence entre 50 cm et 10 mètres.

Selon un mode de mise en oeuvre préféré de la présente invention, ledit mollusque bivalve marin appartient au genre *Crassostrea* ou au genre *Mytilus,* et avantageusement, à l'espèce *Crassostrea gigas* ou à l'espèce *Mytilus galloprovincialis*. Ces deux espèces, qui colonisent à l'état naturel les zones côtières ainsi que les baies et les estuaires, sont euryhalines et peuvent donc être utilisées dans une large gamme de salinité (HIS *et al.*, Mar. Biol., 100, 455-463, 1989). Lorsque l'on souhaite effectuer des bioessais en période hivernale, ou, de manière plus générale, lorsque la température de l'eau au site choisi se situe en dessous des 20°C on choisira de préférence *M. galloprovincialis*, qui est une espèce bradyctique, se caractérisant par la présence dans les populations naturelles de sujets mûrs toute l'année, et dont l'embryogenèse s'effectue encore normalement, quoique lentement, à 10°C.

Le temps nécessaire au développement des oeufs fertilisés jusqu'au stade véligère varie selon le genre concerné ; il est généralement de 24 heures environ pour *Crassostrea* et de 48 heures environ pour *Mytilus.* Il peut toutefois varier en fonction de certaines conditions du milieu ambiant, notamment de la température. Par exemple, dans le cas de bioessais en période hivernale avec *M. galloprovincialis*, le temps de développement peut aller jusqu'à 3 ou 4 jours.

Les autres modalités de mise en oeuvre du procédé conforme à l'invention, à savoir les modalités de fertilisation des oeufs, de récupération des larves, de détermination du taux de mortalité et/ou du taux d'anomalies, ne diffèrent pas de celles habituellement utilisées dans les bioessais en laboratoire connus dans l'art antérieur.

Pour la mise en oeuvre du procédé conforme à l'invention, on emploie un dispositif dans lequel les récipients utilisés sont fixés à un support approprié, lesté ou relié à un lest pour le maintenir en immersion. On peut également placer les récipients dans une cage ou un filet, lestés ou reliés à un lest. Avantageusement, au moins un récipient d'exposition et au moins un récipient témoin sont fixés sur un même support ou placés dans une même cage ou un même filet.

Pour maintenir les récipients à la profondeur souhaitée et permettre leur localisation et leur récupération à la fin de l'essai, ledit support ou ladite cage peuvent en outre être reliés à une bouée ou à un point d'amarrage fixe.

Le récipient d'exposition et le récipient témoin utilisés ont une contenance variant généralement de 20 à 500 ml, de préférence de 50 à 200 ml. La forme desdits récipients peut être très variable ; il peut s'agir par exemple de cylindres ou de parallélépipèdes.

Une ou plusieurs des parois du récipient d'exposition possède(nt) une pluralité d'orifices permettant le passage de l'eau, tout en retenant les oeufs fécondés. Le diamètre d'un oeuf fécondé étant de 60 µm dans le cas des moules et des huîtres, ces orifices n'excéderont pas 40 µm dans leur plus grande dimension.

Avantageusement, la ou lesdites parois sont constituées par un treillage dont la taille des mailles est de 20 à 40 µm, de préférence de 25 à 35 µm. Ledit récipient d'exposition peut également comprendre un sac dont tout ou partie des parois sont en treillage tel que défini ci-dessus. Ledit sac peut être pourvu d'une armature, ou fixé à l'intérieur ou à l'extérieur d'un récipient rigide pourvu d'ouvertures de grande taille permettant à l'eau de circuler librement.

Les matériaux utilisés pour le récipient d'exposition et le récipient témoin doivent être résistants à l'eau de mer et non-toxiques pour les larves. Parmi les matériaux utilisables on citera par exemple le polystyrène, le polyamide, etc.

La présente invention sera mieux comprise à l'aide du complément de description qui va suivre, qui se réfère à un exemple de mise en oeuvre du procédé de bioessai selon l'invention pour l'évaluation *in situ* de la qualité de l'eau.

### EXEMPLE :

### Sites d'expérimentation

La qualité biologique de l'eau du port d'Arcachon a été étudiée. Il s'agit d'un port en eau profonde dont l'échange à la marée est incomplet. Ses 2400 places sont occupées par des navires de plaisance de moyenne taille, la majorité faisant moins de 57 tonneaux ; plus de 80% d'entre eux ne quittent jamais leur amarrage même pendant la saison estivale. Il comprend en outre, dans la partie est, un port de pêche côtière, dont la flotte est composée d'une trentaine de bateaux de plus de dix mètres.

Les sites d'expérimentation suivants ont été choisis :
(A) : à l'entrée du port ;
(B) : dans la partie médiane du port ;
(C) : au fond du port ;
(D) : à l'extérieur de la digue (cet endroit a été choisi comme site témoin non-pollué).

Les dates suivantes ont été retenues compte tenu de la disponibilité de géniteurs mûrs pour effectuer les tests :
- période hivernale (*M. galloprovincialis*), du 10 au 14 janvier.
- période estivale (*C. gigas*) les 15, 27 et 30 Juin et 21 juillet.

### Matériel d'exposition in situ

Les chambres d'exposition sont construites à l'aide de flacons-tubes à prélèvement en polystyrène cristal de 100 ml de capacité (60 cm x 65 cm) à capuchon vissant. Le fond des tubes est ouvert ; dans la paroi verticale de chacun, sont ménagées quatre fenêtres, tout en conservant la structure générale rigide des flacons tubes. Une gaine de même diamètre que le pot, fermée à son extrémité, est cousue à l'aide de fil en polyamide, ou soudée à chaud, dans de la toile à bluter en polyamide de porosité 30 µm. Cette gaine est fixée hermétiquement au niveau de l'ouverture supérieure du pot, à l'aide du capuchon à vis. Cette façon de procéder permet d'éviter toute utilisation de colle, dont les différents types essayés se sont révélés toxiques vis à vis des embryons de bivalves, organismes très sensibles à l'action des micro-polluants organiques. Le temps de rétention de l'eau, dans les pots ainsi préparés, a été estimé par utilisation d'une solution de rouge neutre, à ¼ heure.

### Matériel biologique et tests.

Les tests embryo-larvaires ont été effectués selon un protocole de tests embryo-larvaires en laboratoire décrit par HIS *et al*. (Wat. Res., 31,351-355, 1997).

Les gamètes sont obtenus par stimulation thermique des géniteurs prélevés dans le milieu naturel, dans le cas présent. Après comptage des ovocytes en eau de mer filtrée à 0,2 µm (EMF), on procède aux fécondations avec du sperme fraîchement émis. Au bout de dix minutes, les oeufs fécondés sont répartis, à raison de 600 par chambre, dans les chambres d'exposition préalablement remplies d'EMF au laboratoire.

Les chambres d'exposition sont acheminées dans un récipient contenant de l'EMF sur le site d'observation, et immergées sous un mètre d'eau (3 par site à tester) dans un filet à mailles de 30 mm en nylon. Le filet lesté est suspendu soit à une bouée, soit à l'extrémité d'une des traques du port de plaisance. La mise en place est effectuée dans l'heure qui suit les fécondations.

Lorsque les larves D sont formées à l'issue d'une période d'incubation qui varie de 24 h chez les huîtres à un minimum de 48 h chez les moules, les pots sont ramenés au laboratoire, et les larves sont récupérées sur tamis inox de porosité 32 µm, puis formolées pour observation au microscope (détermination des pourcentages d'anomalies larvaires).

Les anomalies larvaires sont dénombrées sur 100 individus par pot, et comparées avec les anomalies observées au site témoin (site D).

Le test est considéré comme valable si dans les témoins, moins de 20% des véligères sont anormales.

La moyenne des trois répliquats est calculée pour chaque site, avec intervalle de confiance au seuil de sécurité de 95% pour comparaison avec le témoin.

### Résultats

### Période hivernale

Les oeufs fécondés de *M. galloprovincialis* ont été exposés le 10 janvier 1999 en période de mortes eaux (coefficient 41) ; les larves D n'ont été formées qu'après 4 jours. En effet, la température de l'eau de mer a varié de 9,8°C à 10,4°C, pour une salinité moyenne de 31 p. mille. Les pourcentages d'anomalies larvaires dans les témoins (D) se situent en dessous du seuil limite des 20% d'anomalies larvaires, avec une valeur de 14,0 ± 4,2 %. Le pourcentage le plus élevé, relevé au point C n'est que de 19,0 ± 6,2 %.

La qualité biologique des eaux du port apparaît donc normale.

### Période estivale

Quatre séries d'observations ont été réalisées à l'aide d'oeufs fécondés de *C. gigas* pendant l'été 2000.

En juin, la température de l'eau du port a varié de 19°C à 22,4°C et la salinité est restée stable (31 ± 1 p. mille). En juillet, la température a varié de 21°C à 22,4°C, et la salinité était la même que précédemment.

Le 15 juin (coefficient de marée de 70), les pourcentages d'anomalies larvaires sont restés peu élevés à l'exception du point B, dans la partie moyenne du port (27,3 ± 9,7%).

Le 27 juin en période de mortes eaux (coefficient de 48), d'importantes perturbations ont été observées aux point B et C, avec respectivement 52,5 ± 3,5% et 52,0 ± 9,5% d'anomalies larvaires. On notait 27,0 ± 4,2% d'anomalies au point D ; par contre celles-ci n'atteignaient que 14,7 ± 2,0% à l'entrée du port (A).

Le 30 juin (coefficient de marée 70), on observe encore une dégradation marquée de la qualité biologique de l'eau du port, de la zone moyenne (B) à la zone la plus confinée (C), avec les pourcentages respectifs de 41,3 ± 7,6% et 36,4 ± 14,3% ; à l'inverse, à l'entrée (A) et au point D, les valeurs obtenues sont très basses (5,3 ± 0,6% et 6,0 ± 2,5% d'anomalies larvaires seulement).

Enfin le 21 juillet, en période de vives eaux (coefficient de marée de 75), les différences précédemment notées se sont atténuées, avec des valeurs très proches aux points B (25,3 ± 7,8%) et C (24,3 ± 6,0%), toutefois supérieures aux pourcentages notés à l'entrée du port (A, 10,5 ± 0,7% d'anomalies) et au pont D (11,0 ± 4,2%).

### Conclusion

Les pourcentages d'anomalies larvaires au point de référence (D) se situent nettement en dessous de la valeur seuil des 20%, qui est classiquement retenue pour estimer la valeur des tests embryo-larvaires chez les Bivalves marins. Ceci est valable pour les deux espèces utilisées, *C. gigas* et *M. galloprovincialis*. Une exception est toutefois à noter le 27 juin 2000 : les eaux du point D sont de qualité biologique moyenne, le point A pouvant alors servir de référence. Les observations dans ce cas ont été effectuées par très faible coefficient de marée, donc avec un renouvellement réduit de l'eau de mer dans le secteur. C'est d'ailleurs à cette date que les plus mauvais résultats concernant la qualité biologique de l'eau ont été obtenus sur les différents sites étudiés. Il apparaît donc que les tests embryo-larvaires chez les Bivalves marins peuvent être effectués *in situ*.

## Revendications

1. Procédé de détermination de la qualité biologique de l'eau de mer, **caractérisé en ce qu'**il comprend :
a) le transfert d'un nombre défini d'oeufs fertilisés d'un mollusque bivalve marin dans un récipient d'exposition enfermant lesdits oeufs et permettant le passage de l'eau de mer ;
b) l'immersion dudit récipient d'exposition à l'endroit où l'on souhaite déterminer la qualité de l'eau, et son maintien en immersion pendant un temps suffisant pour permettre le développement desdits oeufs jusqu'au stade véligère ;
c) la récupération des larves, et la détermination du taux de mortalité et/ou du taux d'anomalies présentes chez lesdites larves.

2. Procédé selon la revendication 1, **caractérisé en ce qu'**il comprend en outre la réalisation d'au moins un essai témoin, dans lequel on détermine le taux de mortalité et/ou le taux d'anomalies chez des larves véligères développées dans les mêmes conditions, dans une eau de mer non polluée.

3. Procédé selon la revendication 2, **caractérisé en ce que** l'essai témoin comprend les étapes a') b') et c') suivantes :
a') le transfert d'un nombre défini d'oeufs fertilisés d'un mollusque bivalve marin dans un récipient témoin étanche, contenant une eau de mer non polluée ;
b') l'immersion dudit récipient témoin au même endroit que le récipient d'exposition, et son maintien en immersion pendant le même temps ;
c') la récupération des larves dudit récipient témoin, et la détermination du taux de mortalité et/ou du taux d'anomalies présentes chez lesdites larves.

4. Procédé selon une quelconque des revendications 1 à 3, **caractérisé en ce que** ledit mollusque bivalve marin appartient au genre *Crassostrea* ou au genre *Mytilus.*

5. Procédé selon une quelconque des revendications 1 à 4, **caractérisé en ce que** l'immersion s'effectue à une profondeur comprise entre 20 cm et 20 mètres.
